# EUROPÄISCHE PATENTSCHRIFT

(11) **EP 3 969 089 B1**
(45) Veröffentlichungstag und Bekanntmachung des Hinweises auf die Patenterteilung: **29.11.2023**
(21) Anmeldenummer: 20743754.2
(22) Anmeldetag: 18.05.2020
(51) Int. Cl.: A61M 16/04, A61M 25/10, A61F 2/00, A61L 29/00

(54) **BALLON MIT EINEM MEHRLAGIGEN WANDUNGSAUFBAU FÜR DIE GEWEBESCHONENDE NIEDERDRUCK-DICHTUNG VON ÖFFNUNGEN UND HOHLRÄUMEN IM KÖRPER EINES PATIENTEN, INSBESONDERE BEI ZYKLISCH SCHWANKENDEN FÜLLDRUCKWERTEN**
BALLOON WITH A MULTI-LAYER WALL STRUCTURE FOR THE TISSUE-CONSERVING LOW-PRESSURE SEALING OF OPENINGS AND CAVITIES IN THE BODY OF A PATIENT, ESPECIALLY IN THE CASE OF CYCLICALLY FLUCTUATING FILLING PRESSURE VALUES
BALLONNET PRÉSENTANT UNE STRUCTURE DE PAROI MULTICOUCHE POUR L'ÉTANCHÉITÉ À BASSE PRESSION, RESPECTUEUSE DES TISSUS, D'OUVERTURES ET DE CAVITÉS CORPORELLES D'UN PATIENT, EN PARTICULIER POUR DES VALEURS DE PRESSION DE REMPLISSAGE À FLUCTUATION CYCLIQUE

(30) Priorität: 16.05.2019 DE 102019003482
(43) Veröffentlichungstag der Anmeldung: 23.03.2022
(73) Patentinhaber: Creative Balloons GmbH, 68753 Waghäusel (DE)
(72) Erfinder: GÖBEL, Fred, 67346 Speyer (DE)
(74) Vertreter: Küchler, Stefan
(86) Internationale Anmeldenummer: PCT/IB2020/054684
(87) Internationale Veröffentlichungsnummer: WO 2020/230111

(56) Entgegenhaltungen:
- EP-A1- 0 214 721
- US-A- 5 490 839
- US-A1- 2008 092 902
- US-A1- 2010 006 102
- US-A1- 2015 290 434

## Beschreibung

Die Erfindung richtet sich auf eine ballonartige Struktur zur Positionierung innerhalb eines Hohlraums im menschlichen oder tierischen Körper, beispielsweise innerhalb eines Lumens oder eines sonstigen Binnenraums, insbesondere als Bestandteil eines Katheters, derart, dass der besagte Hohlraum zum einen möglichst vollständig, also ohne verbleibenden residualen Raum ausgefüllt wird, dabei aber in seiner Form weitestgehend erhalten bleibt, bzw. durch den Ballonkorpus nicht deformiert wird.

Katheteranwendungen im Körper eines Patienten erfordern in vielen Fällen ballonartige Komponenten, die ein Lumen oder einen Binnenraum in dichtender Weise verschließen oder raumfüllend tamponieren, wobei die dichtende und/oder tamponierende Funktion des jeweiligen Ballons auch dann erhalten bleibt, wenn sich die Querschnittfläche des zu dichtenden Lumens, bzw. sich das Volumen des zu tamponierenden Raumes, bedingt durch die physiologische Funktion der jeweiligen Struktur oder bedingt durch Bewegungen des Körpers, in intermittierender oder beispielsweise zyklischer Weise schwankt. Ferner soll bei langfristig im Körper platzierten Ballonkomponenten, die vom Ballon auf die anliegenden Gewebe und Strukturen übertragene Kraft dabei in einem Bereich liegen, der die Perfusion erhält und druckbedingte Läsionen ausschließt.

Grundsätzliche Lösungswege für das Problem einer effizienten, gewebeverträglichen Dichtung eines Organs oder Hohlraumes wurden bereits in der EP 1 061 984 A1 in konkretem Bezug auf die Dichtung der Luftröhre (Trachea) gegen Sekrete des Rachenraumes vorgestellt, Die besondere Qualität der dort beschriebenen trachealen Dichtung beruht im Wesentlichen auf der Verwendung sehr dünnwandiger Ballonfolien aus Polyurethan (PUR).

Der bei der Herstellung ausgeformte Umfang des Ballons (Cuff) überschreitet dabei den Umfang des zu dichtenden trachealen Abschnitts, was bei der Befüllung des residual bemessenen Ballons innerhalb der zum Ballon relativ kleineren Trachea die Ausbildung eines typischen Einstülpungsmusters der Ballonhülle verursacht. Das residuale, also entlang des Umfangs des Ballons überschüssige Ballonmaterial formiert sich dabei in radialen, d.h., dem Verlauf von Radspeichen folgenden Invaginationen. Diese bilden an ihrem blinden, d.h. zu dem Zentrum des Ballons gerichteten Ende, spezifische, der Längsachse des Ballons folgende, kanalartige Formationen aus, die den freien Durchfluss von Sekreten bzw. Flüssigkeiten erlauben. Die Ausbildung derartiger Einstülpungen ist für das Niederdruckverhalten und die Aufrechterhaltung der Perfusion der anliegenden Strukturen entscheidend. Das Prinzip der Invagination einer residual bemessenen Ballonhülle stellt sicher, dass für den Verschluss des jeweiligen Lumens oder Raumes die Hülle nicht in den Zustand einer kraftintensiven Aufdehnung überführt werden muss, sondern sich in das jeweilige Lumen oder den betreffenden Binnenraum des Patienten spannungslos hinein "falten" kann, was bereits bei Ballonfülldruckwerten möglich ist, die den jeweiligen lokalen Druck nur geringfügig überschreiten. Die jeweilige Form und Größe des Lumens oder sonstigen Hohlraumes des Patienten bleiben so erhalten.

Die EP 1 061 984 A1 beschreibt für eine aus PUR bestehende, residual bemessene Ballonhülle, einen bestimmten Wandungsstärkenbereich, nämlich von 5 bis 20 µm, bei dem sich bei in situ platzierter Ballonhülle kanalartige Formationen einstellen, deren innerer Durchmesser den freien Durchfluss von Sekret hemmt bzw. auf das Sekret einen kapillaren, staseartigen Effekt hat. Die Durchmesser der Kanälchen werden in der EP 1 061 984 A1 mit kleiner als 0,11 mm, bzw. vorteilhaft kleiner als 0,05 mm beschrieben.

Der sich im Verlauf einer trachealen Beatmungssituation jeweils einstellende Durchmesser der sich endständig an den Invaginationen der Cuff-Hülle ausformenden Kanälchen ist neben der materialspezifischen Ausführung der Hülle selbst im Wesentlichen vom jeweiligen, aktuell im Ballon herrschenden Fülldruck abhängig. Verringert sich der Fülldruck, beginnen sich die kanalartigen Strukturen zu weiten, wobei sich die Kanälchen, vom jeweiligen blinden Ende der Invaginationen ausgehend, zur trachealseitigen Basis oder Mündung der Invaginationen hin sukzessive öffnen. Bei einem weiteren Abfall des Fülldrucks im Ballon schließt sich die Invagination zur trachealen Schleimhaut hin vollständig auf und geht in eine Konfiguration mit einem in radialer Richtung etwa U- oder auch W-förmigem Verlauf über. Abhängig von dem jeweiligen Ausmaß der Öffnung der Invaginationen bzw. der sich dabei effektiv einstellenden Querschnittsfläche der Kanälchen resultiert das zu einem bestimmten Zeitpunkt wirksame Dichtungsvermögen des Cuffs.

Im Rahmen einer derartig dichtenden oder tamponierenden Ballontechnologie stellt sich eine besondere Herausforderung bei der Dichtung von Organen oder Räumen, deren Binnendruck zyklische Schwankungen durchläuft, wie dies beispielsweise bei der Trachea (Luftröhre) oder dem Ösophagus (Speiseröhre) der Fall ist. Beide Strukturen sind im Thorax kontinuierlichen, zyklischen Druckschwankungen ausgesetzt, die durch die Eigenatmung des Patienten generiert werden. Sowohl bei der nichtunterstützten Atmung als auch bei der maschinell unterstützten Atmung des Patienten korrespondieren die jeweilig generierten thorakalen Drucke mit dem Fülldruck des tracheal dichtenden Cuffs.

Kommt es während der Einatmung (Inspiration) des Patienten zu einem Abfall des im Thoraxraum herrschenden thorakalen Drucks, überträgt sich diese passagere Druckverminderung auf die tracheale und ösophageale Wandung, was wiederum zu einer Druckreduktion in entsprechenden, tracheal oder ösophageal positionierten Ballonstrukturen führt. Im Moment der Entlastung der trachealen oder ösophagealen Wandung weiten sich die Querschnittflächen der endständigen, kanalartigen Formationen zeitsynchron auf, wodurch es über die Phase der Weitung zu einer vermehrten Passage für Sekrete und Flüssigkeiten kommt, die bis hin zu einem vollständigen, Dichtungsverlust des Ballons und einer bolusartigen Aspiration führen kann.

Auf die besondere Abhängigkeit der Dichtungseffizienz von Trachealtuben-Cuffs von der thorakalen Atemarbeit des Patienten wird u.a. von Badenhorst und Kollegen ["Changes in cuff pressure during respiratory support", C.H, Badenhorst, Critical Gare Medicine 1987, 15;4, 300-302] hingewiesen. Badenhorst beschreibt bei einzelnen Patienten Cuff-Druckwerte, die, von ca. 20 mbar ausgehend, bis in den subatmosphärischen Bereich reichen, und somit den im Thorax des atmenden Patienten herrschenden intra-thorakalen Druckwerten weitgehend folgen.

In der aktuellen Literatur zum Dichtungsverhalten residual bemessener Trachealtuben-Cuffs, sogenannter high-volume, low-pressure Cuffs, wird die jeweilige Fähigkeit bestimmter Cuff-Typen zur Gewährleistung einer wirksamen Sekretdichtung an statischen Betrachtungsmodellen ermittelt. So wird beispielsweise in einer von Bassi und Kollegen durchgeführten Untersuchung ["An in vitro study to assess determinant features associated with fluid sealing in the design of endotracheal tube cuffs and exerted tracheal pressures"; Bassi et al.; Critical care Medicine, 2013; 41:518-526] ein starres Rohr mit verschiedenen Trachealtuben intubiert, und im Anschluss daran, oberhalb des mit Fülldruck beaufschlagten Trachealtuben-Cuffs eine Wassersäule installiert. Die im statischen Modell festgestellte Leckage liegt bei herkömmlichen, PVC-basierten Cuff-Typen, bei einem anliegenden, empfohlenen Fülldruck von 30 mbar bereits im Bereich von mehreren Millilitern pro Minute. Lediglich PUR-basierte, sehr dünnwandige Cuff-Typen ermöglichen auch bei einer Reduktion des Fülldrucks auf 15 mbar noch eine zuverlässige Dichtungswirkung. Bei Fülldrucken unterhalb von 15 mbar zeigen allerdings auch PUR-Ballons mit Wandungsstärken von weniger als 20 µm eine beginnende Weitung der sekretleitenden, kanalartigen Formationen, wobei sich, wie bei den deutlich dickwandiger hergestellten PVC-Cuffs, die Querschnitte der Kanälchen vom blinden Ende der jeweiligen Invagination zu deren Basis hin tropfenförmig öffnen und schließlich vermehrt Sekrete und Flüssigkeiten passieren lassen.

Zusammenfassend sind statische Modelle nicht in der Lage die beschriebenen, zyklisch schwankenden Änderungen der trachealen Querschnittfläche als skalierbaren Faktor aufzunehmen. Sie sind somit ungeeignet, die klinisch wirksame Qualität einer ballon-basierten Dichtung in der Trachea unter Druckschwankungen abzubilden.

US5490839 A beschreibt einen aufblasbaren Ballon für einen Katheter, wobei der Ballon eine Beschichtung enthält, die bewirkt, dass der Ballon beim Entleeren eine vorbestimmte, flache Konfiguration, wie z.B. eine dreifach gefaltete Konfiguration, annimmt. Der Ballon hat eine Wand, die mit einer äußeren Polymerbeschichtung versehen ist. Die Beschichtung wird aufgebracht, während sich der Ballon in der vorbestimmten Niedrigprofilkonfiguration im entleerten Zustand befindet, so dass die Beschichtung nach dem Aufblasen den Ballon dazu zwingt, in die Niedrigprofilkonfiguration zurückzukehren, wenn der Ballon entleert wird.

US2010/006102 A beschreibt eine Manschette einer Beatmungsvorrichtung, insbesondere einer Tracheostomiekanüle oder eines Endotrachealtubus, wobei die Manschette einen Beatmungstubus umgreift und abgedichtet an diesem befestigt ist und die Manschette aus einer inneren Folie und einer äußeren Folie besteht, wobei die innere und die äußere Folie voneinander getrennt sind. Die äußere Folie beseht aus einem elastisch leicht dehnbaren Material und die innere Folie besteht aus einem Material mit geringerer elastischer Dehnung, wobei die innere Folie mit Übermaß hergestellt ist.

Die Erfindung wird durch den unabhängigen Anspruch 1 definiert. Die abhängigen Ansprüche definieren bevorzugte Ausführungsformen.

Die Aufgabe der Erfindung besteht darin, dass, um die Effizienz dichtender Ballonkomponenten unter den Bedingungen organsynchron zyklischer Schwankungen des Ballonfülldrucks zu verbessern, neuartige Cuff-Bautypen geschaffen werden sollen, die auch dann ein effizientes Dichtungsverhalten gewährleisten, wenn der Fülldruck des Ballons in kontinuierlicher Weise über eine von 30 bis 5 mbar reichende Druckamplitude schwankt.

Die Lösung dieser Aufgabe gelingt im Rahmen einer gattungsgemäßen, ballonartigen Struktur dadurch, dass die Ballonhülle aus einem mehrlagigen Ballonfolienmaterial besteht, wobei wenigstens eine Lage aus einem elastisch verformbaren Polyurethan (PUR) besteht und wenigstens eine andere Lage aus einem nicht elastischen Material wie beispielsweise Polyvinylchlorid (PVC), wobei die wenigstens eine PUR-Lage aus einem thermoplastischen PUR von einem Typ mit einer Wasseraufnahme nach DIN ISO 62 von 5% oder weniger besteht, bevorzugt mit einer Wasseraufnahme nach DIN ISO 62 von 2% oder weniger, wobei die Verbindung der mehreren Lagen der Ballonhülle durch Koextrusion der verschiedenen Lagen hergestellt ist.

Die vorliegende Erfindung beschreibt damit Lösungswege zur Verbesserung des Dichtungsverhaltens von dichtenden und/oder tamponierend wirkenden, weichfolienartigen Ballonkörpern, die in eigenständig motilen oder auch in passiv motilen Organen oder Körperräumen eingebracht und dort dauerhaft positioniert werden können, auch und gerade dann, wenn der jeweilige Binnendruck im Organ und/oder die jeweilige Gestalt des Raumes in intermittierender, in kontinuierlicher, oder insbesondere auch in zyklisch schwankender Weise, Veränderungen durchlaufen. Die Wandungen der erfindungsgemäßen Ballonkörper weisen dabei einen spezifischen, mehrlagig kombinierten Aufbau aus sich nicht-elastisch verformenden, und sich elastisch verformenden Materiallagen auf.

Dabei umfasst das Ballonfolienmaterial eine oder mehrere Lagen eines sich elastisch verformendem Materials, mit einer oder mehreren Lagen eines sich plastisch verformendem, nicht-elastischen Materials, wobei die nichtelastische Lage den Aufrichtungseigenschaften der elastischen Lage bei flächigen Faltungen oder Verbiegungen des Ballonfolienmaterials entgegenwirkt.

Bei entsprechend mehrlagig aufgebauten Ballonkomponenten, die bei der Platzierung und Beaufschlagung mit Fülldruck in situ faltenartige Invaginationen der residualen Ballonhülle ausbilden, werden die elastischen Aufrichtungs- und Öffnungseigenschaften der querschnittlich ösen- bzw. kanalartigen Formationen durch den Materialverbund mit einem nicht-elastischen Material reduziert, so dass sich bei intermittierenden oder zyklischen Schwankungen des Ballonfülldrucks, in Relation zu einlagigen Ballonhüllen gleicher Wandungsstärke aus elastischem Material, wie beispielsweise PUR, innerhalb der ösen- und kanälchenartigen Strukturen kleinere und unter den Bedingungen einer Schwankung des Fülldrucks insgesamt kleinere und in einem geringeren Umfang schwankende Querschnittflächen der endständigen, sekretleitenden Querschnittflächen formieren. Besonders vorteilhaft verhalten sich dabei solche Kombinationen, bei denen die Wandungsstärke der verbauten PUR-Lage auf einen möglichst kleinen Anteil an der Gesamtwandungsstärke der Ballonhülle reduziert wird.

Wird der Ballon in einem Blasformungsverfahren aus einem zuvor hergestellten, mehrlagigen Rohschlauchmaterial ausgeformt, wirkt die anteilige PUR-Lage im Formungsverlauf stabilisierend und ermöglicht auch bei besonders dünnwandig ausgeformten Ballonhüllen eine gute Symmetrie des Ballonkorpus, wobei der umzuformende Rohschlauch bei der Beaufschlagung mit Blasdruck sukzessive über eine gleichmäßig geformte Spindelform in eine gleichmäßig geformte sphärische Ballonform übergeht, sich schließlich in die jeweilige Blasform hinein expandiert und deren Form annimmt. Die elastischen Eigenschaften von PUR ermöglichen, über eine gleichmäßige Symmetrie hinaus, die qualitativ stabile Formung von Ballonkomponenten mit extrem niedrigen Gesamtwandungsstärken, im Bereich von beispielsweise 5 bis 20 Mikrometern, und statten diese darüber hinaus in der Anwendung mit einer hohen mechanische Belastbarkeit, Punktionsbeständigkeit und einer in der Regel sehr guten Formbeständigkeit bei passageren und auch dauerhaften Überschreitungen der jeweiligen Arbeitsfülldrucke aus.

Die erfindungsgemäße Kombination einer oder mehrerer PVC-basierter Materiallagen mit einer den Ballonkörper mechanisch stabilisierenden PUR-Lage ist ferner für die Reduktion von PUR-typischen Durchlässigkeitseffekten von Wassermolekülen von Vorteil. Wird eine wasserdurchlässige PUR-Lage mit einer PVC-Lage verbunden, welche deutlich bessere Barriere-Eigenschaften gegen polare Substanzen als PUR aufweist, kann insbesondere die Auskondensation und Akkumulation von Wasser im Ballon reduziert werden,

Um die Gesamtwandungsstärke einer erfindungsgemäß mehrlagig aufgebauten Ballonfolie möglichst niedrig, d.h. in einem bevorzugten Bereich von 5 bis 30 Mikrometern zu halten, schlägt die Erfindung den Einsatz von PUR-Typen vor, die durch eine möglichst geringe Quellungsneigung der Ballonwandung durch die Aufnahme von Wassermolekülen charakterisiert sind. Derartige Quellungseffekte sind vor allem bei nicht-thermoplastischen Polyurethanen bekannt. Die Erfindung verwendet daher bevorzugt thermoplastische PUR-Typen mit einer Wasseraufnahme nach DIN ISO 62 im exponierten, wässrigen Milieu, von kleiner 4%, bevorzugt kleiner 2%, wie beispielsweise die Typen der Produktfamilie "Pellethane 2363" der Firma Lubrizol Inc. oder der Produktfamilie "Elastollan 1100" der Firma BASF AG.

Weitere Merkmale, Eigenschaften, Vorteile und Wirkungen auf der Basis der Erfindung ergeben sich aus der folgenden Beschreibung einiger bevorzugter Ausführungsformen der Erfindung sowie anhand der Zeichnung. Hierbei zeigt:
- Fig.1: einen erfindungsgemäßen Aufbau einer Ballonhülle aus zwei kombinierten Materiallagen in einer schematischen Darstellung;
- Fig. 2a: eine sekretleitende, kanalartige Formation, wie sie sich bei im Umfang residual bemessenen, dichtenden und/oder tamponierenden Ballonkörpem in einem relativ zum Ballon kleineren Lumen entwickelt, in einem transversalen Anschnitt;
- Fig, 2b: eine entsprechende, sekretleitende, kanalartige Formation, die sich bei reduziertem Fülldruck tropfenförmig vergrößert, und bei weiterer Reduktion des Fülldrucks U-förmig öffnet;
- Fig. 3: einen Trachealtuben-Cuff, wobei die kanalartigen Formationen den Ballonkörper von einer Stirnseite zur anderen überbrücken, in einer schematischen Darstellung;
- Fig, 4: eine 2-lagige Ausführungsform einer Ballonwandung, wobei die tragende PUR-Lage mit einer wasserdampfdichten Barriere-Lage aus PVDC kombiniert ist;
- Fig. 5: eine 3-lagige Ausführungsform einer Ballonwandung, wobei die tragende PUR-Lage mit einer mittigen Barriere-Lage aus PVDC und/oder EVOH und mit einer die elastischen Aufrichtungseigenschaften von PUR dämpfenden PVC-Lage kombiniert ist; sowie
- Fig. 6: einen qualitativen Vergleich zwischen zwei aus elastischem PUR ausgeformten Ballontypen, wobei einer der Bautypen mit einer die elastischen Eigenschaften von PUR modifizierenden Lage von PVC kombiniert ist.

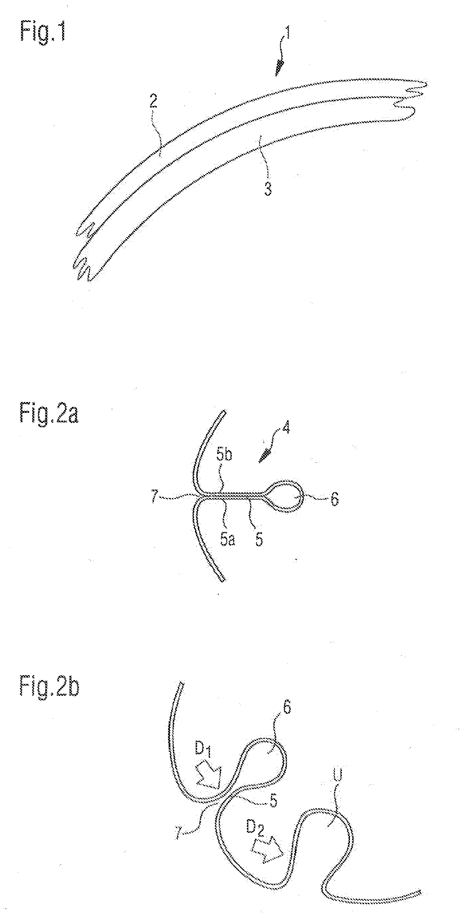

Fig. 1 zeigt in schematischer Ausführung einen beispielhaften, erfindungsgemäßen, 2-lagigen Aufbau einer Ballonwandung 1, wobei vorzugsweise die äußere, dem jeweiligen Lumen oder Hohlraum zugewandte Materiallage 2 aus thermoplastischem PUR des Typs Elastollan 1100 mit einer Härte nach Shore von 90A besteht und eine anteilige Wandungsstärke von 5 bis 10 Mikrometern aufweist, Bevorzugt besteht die dem Binnenraum des Ballons 12 zugewandte Materiallage 3 aus einem PVC mit einer Härte nach Shore von 70A und weist eine anteilige Wandungsstärke von 15 bis 20 Mikrometern aus. Die beiden Polymere werden vorzugsweise direkt, also ohne eine haftungsvermittelnde Zwischenlage, durch einen Koextrusionsprozess, in fest aneinander haftender Weise, flächig verbunden hergestellt. Die 15 bis 20 Mikrometer starke PVC-Lage wirkt zum einen der elastischen Aufrichtung der zu einer ösenartigen Formation gefalteten anteiligen PUR-Lage in dämpfender, die Geschwindigkeit und das Ausmaß der Aufrichtung reduzierender Weise entgegen. Zum anderem reduziert die anteilige PVC-Lage den Durchtritt bzw. die Migration polarer Substanzen durch die beschriebene PUR/PVC-Lagenkombination, und reduziert somit unerwünschte Kondensations- und Akkumulationseffekte von Flüssigkeit im Binnenraum des Ballons, insbesondere von Wasser,

Im Rahmen der Erfindung können die aus PVC und PUR bestehenden Wandungslagen auch derart innerhalb des Lagerverbundes angeordnet werden, dass sich sie PVC-Lage auf der Ballon-Außenseite befindet.

Neben 2-lagigen Ballonwandungen sind beispielsweise auch 3-lagige Ausführungsformen möglich, wobei die PUR-Lage bevorzugt zwischen zwei PVC-Lagen in sandwichartiger Weise eingefasst wird. Bei einer Gesamtwandungsstärke von 30 Mikrometern kann die Verteilung der Einzellagen beispielsweise außen 12 µm PVC, mittig 6 µm PUR und außen 12 µm PVC aufweisen. Diese Ausführungsform ist besonders vorteilhaft bei der Begrenzung unerwünschter Migrationseffekte polarer Substanzen, wie zum Beispiel Wasser.

Fig. 2a zeigt im Schema den transversalen Anschnitt einer sekretleitenden Invagination 4, wie sie sich bei einem residual, d.h. auf ein Übermaß ausgeformten, dichtenden und/oder tamponierenden Ballonkörper bei der Platzierung innerhalb eines in Relation zu dem residual bemessenen Ballon kleineren Lumens oder Raums durch Einstülpung der überschüssigen Ballonwandung faltenartige Strukturen entwickeln. Insbesondere bei zyklisch wechselnden Fülldruckwerten innerhalb des Ballons kommt es im Anwendungsverlauf zu einer typischen, vom Umfang zum Zentrum des Ballons gerichteten, radspeichenartigen Anordnung derartiger Einstülpungen.

Die Invaginationen weisen dabei jeweils einen stegartigen, flächig geschlossenen Anteil 5 auf, während sich am blinden, zum Ballonzentrum gerichteten Ende jeder Invagination eine ösenartige Formation 6 ausbildet. Im Bereich der sich formierenden Öse schlägt die Wandung der Ballonhülle um 180 Grad um, wobei durch die elastischen Aufrichtungseigenschaften der in die Wandung integrierten PUR-Lage eine ausgeprägte, öffnende Wirkung auf die ösenartige Formation generiert wird. Abhängig von der Größe der Querschnittsfläche der jeweiligen ösenartigen Formation, sowie von einer größtmöglichen Vermeidung bzw. Reduktion von zyklischen Kalibersprüngen der Öse, resultiert die jeweilige, zu einem bestimmten Zeitpunkt effektive Dichtungswirkung des Ballons. Kleine Querschnittflächen der Öse wirken sich aufgrund von Kapillareffekten auf Sekrete, die sich innerhalb der Öse befinden, in flusshemmender Weise bis hin zur vollständigen Stase des Sekretes bzw. des Öseninhaltes aus Der den freien Durchfluss von Sekret hemmende Effekt geht mit zunehmender Weitung bzw. Vergrößerung der Querschnittfläche der Öse verloren.

Die dichtungsrelevante Querschnittsfläche der ösenartigen Formation 6 wird neben der jeweiligen Eigenschaft zur elastischen Aufrichtung der ösenartig umgeschlagenen Ballonwandung durch den jeweils aktuell im Ballon herrschenden Fülldruck bestimmt, der insbesondere den beiden Wandungslagen 5a und 5b des stegartigen Anteils 5 der Invagination 4 anliegt, und diese flächig, in dicht schließender Weise aneinander presst, wobei im Bereich des Umschlags der beiden Wandungslagen, d.h. an dem blinden Ende der betreffenden Invagination, ein offenes Lumen verbleibt.

Die Gesamtwandungsstärke eines erfindungsgemäß ausgeführten Ballons soll vorzugsweise 30 µm nicht überschreiten. Bei der bevorzugten Ausführung des Ballons liegt das Verhältnis der anteiligen Wandstärke der PUR-Lage zur anteiligen Wandstärke der PVC-Lage zwischen 1 : 2 und 1 : 4, bevorzugt bei einem Verhältnis von 1 : 3.

Kommt es beispielsweise bei einer spezifischen, wie in Fig. 1 beschriebenen Lagenkombination zu einer zyklischen, durch die Eigenatmung des Patienten generierten Schwankung des Fülldrucks im Ballon zwischen 30 und 5 mbar, so resultiert dies in einem Zuwachs der die Dichtungseffizienz des Ballons bestimmenden Querschnittfläche der Ösen von 10% bis 25%, in der Regel jedoch von nicht mehr als 20%. Die größten, innerhalb einer jeweiligen ösenartigen Formation gemessenen Ösendurchmesser erfindungsgemäß hergestellter Ballons liegen bei einem durchgängigen Fülldruck von 30 mbar bei ca. 30 bis 120 µm, bevorzugt bei ca. 40 bis 80 µm.

Bei zyklischen Schwankungen des Ballonfülldrucks von beispielsweise 20 Wechseln pro Minute und Druckamplituden oder Druckextremwerten zwischen 30 und 5 mbar, bleiben die Dichtungseigenschaften des erfindungsgemäßen Ballons, zum Beispiel bei der konkreten Verwendung für die tracheale Sekretdichtung, weitestgehend erhalten. Pumpartige, der Eigenatmung des Patienten synchron folgende, zyklisch melkende Effekte auf die ösenartige Formation 6 bzw. auf die sich aus den Ösen ausformenden Kanäle, wie diese in der medizinischen Literatur bei dickwandigen, einlagigen, PVC-basierten Cuffs der Wandungsstäre von 70 bis 120 Mikrometer beschrieben sind, bleiben bei einem erfindungsgemäß ausgeführten Trachealtuben-Cuff weitgehend aus.

Fig. 2b zeigt eine der Fig. 2a entsprechende, ösenartige Formation 6 im Zustand einer relativ zu Fig. 2a reduzierten Fülldrucksituation, Unterschreitet der Fülldruck einen gewissen dichtungskritischen Fülldruck D1, beginnt sich der Eintrittsbereich 7 an der Basis der Invagination 4 zu öffnen, und die ösenartige Formation 6 weitet und verlängert sich, vom blinden, inneren Ende der Invagination beginnend, zur äußeren Basis der Invagination hin fortschreitend. Das stegartige, dicht schließende Segment 5 der Invagination verkürzt sich entsprechend. Bei einem weiteren Abfall des Fülldrucks auf einen Wert D2 öffnet sich das stegartige Segment 5 vollständig, und die Invagination geht in eine flächig offene U-Form U über.

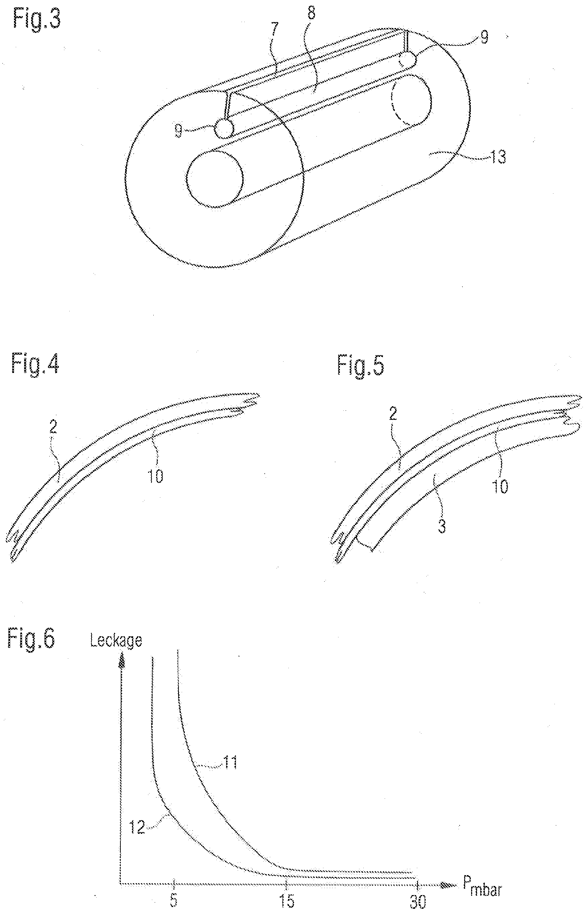

Fig. 3 zeigt schematisch, am Beispiel eines zylindrischen Dichtungsballons, wie dieser beispielsweise als sekretdichtender Trachealtuben-Cuff zur Anwendung kommt, kanalartige Formationen 8, die aus den ösenartigen Umschlagformationen 6 am blinden Ende. der jeweiligen Invaginationen hervorgehen. Die kanalartigen Formationen erstrecken sich dabei von einer Stirnseite 9 des Ballonzylinders zur gegenüberliegenden Stirnseite in durchgängiger Weise, bzw. nehmen bei kontinuierlicher Belastung mit zyklisch wechselnden Fülldrucken in vielen Fällen eine in etwa parallele Ausrichtung zur Zylinderachse des Ballons an, und ermöglichen somit die Leckage von Flüssigkeiten oder Sekreten von der einen zu der anderen Stirnseite des ein Lumen oder einen Binnenraum eines Patienten in dichtender Weise verschließenden oder raumfüllend tamponierenden Ballons,

Fig. 4 zeigt eine besondere, 2-lagig ausgeführte Ballonwandung, wobei die den Ballon stabilisierende PUR-Lage 2 mit einer wasserdampf- und gasdichten Barriere-Lage 10 aus PVDC kombiniert ist. Die PVDC-Lage 10 kann sowohl zur Außenseite des Ballons als auch zu dessen Innenseite hin orientiert sein, PVDC wirkt bereits in sehr dünnen Lagenstärken sehr effizient wasser- und gasdichtend. Die vorgeschlagene Kombination bietet somit die Grundlage für die Herstellung besonders vorteilhafter, niedriger Gesamtwandungsstärken des Ballons im Bereich von 10 bis 15 Mikrometern, wie sie im Sinne einer möglichst kleinen bzw. möglichst konstanten, nicht alternierenden Querschnittsfläche der ösenartigen Formation 6 vorteilhaft sind. Die PUR-Lage 2 weist dabei eine Lagenstärke von beispielsweise 5 Mikrometer auf, die PVDC-Lage 10 dagegen eine Stärke von beispielsweise 5 bis 10 Mikrometer.

Fig. 5 zeigt eine besondere, 3-lagige Ausführungsform einer Balionwandung, wobei die elastische PUR-Lage 2 mit einer mittig angeordneten, gas- und wasserdampfdichte Barriere-Lage 10, beispielsweise aus PVDC oder alternativ auch aus EVOH, und einer die elastischen Aufrichtungseigenschaften von PUR erfindungsgemäß dämpfenden Lage 3, bevorzugt aus PVC niedriger Shorehärte, kombiniert ist, Bei einer Gesamtwandungsstärke von beispielsweise 25 Mikrometer weist die PUR-Lage 2 dabei eine Lagenstärke von beispielsweise 5 Mikrometern auf, die gas- und wasserdampfdichte Barriere-Lage Lage 10 aus bspw. PVDC eine Stärke von 5 Mikrometer, und die dämpfende Lage 3 aus bspw. PVC eine anteilige Lagenstärke von 15 Mikrometer auf.

Fig. 6 veranschaulicht anhand zweier Graphen 11, 12 in qualitativer Weise, wie sich die im Ballonbinnenraum herrschenden Fülldrucke auf die für die Dichtungseffizienz der jeweiligen, dichtenden oder tamponierenden Katheter- oder Device-Anwendung relevante Querschnittfläche der ösenartigen Formation 6 auswirken. In einer vergleichenden Näherung wird gemäß einem Graph 11 ein residual bemessener, radiale Invaginationen der residualen Ballonhülle ausformender, einlagig aus PUR hergestellter Ballon mit einer Wandungsstärke von 15 Mikrometer, gefertigt aus Material des Typs "Elastollan 1190A", einem Graph 12 entsprechend einem residual ausgeformten und bemessenen Ballon aus zweilagigem Material bestehend aus einer erfindungsgemäßen Kombination einer PUR- und einer PVC-Lage, mit einer Gesamtwandungsstärke von 20 Mikrometer, wie in Fig.1 in beispielhafter Weise für die Technologie beschrieben, gegenübergestellt. Bei ca. 20 zyklischen Schwankungen pro Minute, die jeweils den Druckbereich zwischen 30 mbar bis 15 mbar durchlaufen, weisen beide Ballontypen einen vergleichbar effizienten Dichtungseffekt auf, der einer nahezu vollständigen Dichtung entspricht. Reichen die unteren Werte der Druckschwankungen jedoch in den Bereich von 15 bis 5 mbar, trennen sich die beiden Graphen, wobei sich die Querschnittsfläche der Öse bei der Variante 11 im Vergleich zur Variante 12 um ca. 10 bis 25% vergrößert. Bei dem einlagigen Ballon gemäß Graph 11 geht die Dichtung in einem Druckbereich unterhalb von 5 mbar gänzlich verloren, was bei dem erfindungsgemäßen, mehrlagigen Ballon aus einer erfindungsgemäßen Kombination einer PUR- und einer PVC-Lage erst unterhalb von etwa 3 mbar der Fall ist.

### Bezugszeichenliste

- 1: Ballonwandung
- 2: Materiallage
- 3: Materiallage
- 4: Invagination
- 5: stegartiger Anteil
- 6: ösenartige Formation
- 7: Eintrittsbereich
- 8: kanalartige Formation
- 9: Stirnseite
- 10: Barriere-Lage
- 11: Graph
- 12: Graph
- 13: Ballonhülle
- D1: Druckwert
- D2: Druckwert
- U: U-Form

## Patentansprüche

1. Ballonartige Struktur mit einem Ballonkorpus und einer Ballonhülle (13), zur Positionierung innerhalb eines Hohlraums im menschlichen oder tierischen Körper, beispielsweise innerhalb eines Lumens oder eines sonstigen Binnenraums, insbesondere als Bestandteil eines Katheters, derart, dass der besagte Hohlraum zum einen möglichst vollständig, also ohne verbleibenden Raum ausgefüllt wird, dabei aber in seiner Form weitestgehend erhalten bleibt, bzw. durch den Ballonkorpus nicht deformiert wird, wobei die Ballonhülle (13) aus einem mehrlagigen Ballonfolienmaterial besteht, **dadurch gekennzeichnet, dass** wenigstens eine Lage (2) aus einem elastisch verformbaren Polyurethan (PUR) besteht und wenigstens eine andere Lage (3) aus einem nicht elastischen Material wie beispielsweise Polyvinylchlorid (PVC), wobei die wenigstens eine PUR-Lage (2) aus einem thermoplastischen PUR von einem Typ mit einer Wasseraufnahme nach DIN ISO 62 in der am Anmeldetag gültigen Version: dritte Auflage aus dem Jahr 2008 von 5% oder weniger besteht, bevorzugt mit einer Wasseraufnahme nach DIN ISO 62 in der am Anmeldetag gültigen Version: dritte Auflage aus dem Jahr 2008 von 2% oder weniger, wobei die Verbindung der mehreren Lagen (2,3,10) der Ballonhülle (13) durch Koextrusion der verschiedenen Lagen (2,3,10) hergestellt ist.

2. Ballonartige Struktur nach Anspruch 1, **dadurch gekennzeichnet, dass** eine Lage (2) aus sich elastisch verformendem PUR-Material mit einer Lage (3) aus sich plastisch verformendem Material kombiniert ist, insbesondere aus plastisch verformbarem PVC.

3. Ballonartige Struktur nach Anspruch 1 oder 2, **dadurch gekennzeichnet, dass** sich bei der in situ-Platzierung eines auf ein residuales Maß, d.h. mit einem entlang des Ballonumfangs überschüssigen Ballonmaterial ausgeformten Ballonkorpus typische, in des Balloninnere invaginierte Einstülpungen der überschüssigen, residualen Ballonhülle formieren, vorzugsweise wobei die in des Balloninnere invaginierten Einstülpungen querschnittlich ösenartigen Umschlagformationen aufweisen, die sich vorzugsweise in Längsrichtung des Ballons, also zwischen dessen distaler und proximaler Stirnseite (9), als kanalartige Formationen (9) erstrecken bzw. fortsetzen, insbesondere wobei die querschnittlich ösenartigen Umschlagformationen (6), die sich vorzugsweise in Längsrichtung des Ballons als kanalartige Formationen (9) erstrecken bzw. fortsetzen bei einem Fülldruck des Ballons von 30 mbar einen Öffnungsdurchmesser zwischen 30 µm und 120 µm aufweisen, vorzugsweise einen Ösendurchmesser zwischen 40 µm und 80 µm, beispielsweise wobei der Öffnungsdurchmesser der ösenartigen oder kanalartigen Formation (8) reduziert ist gegenüber dem Öffnungsdurchmesser einer ösenartigen oder kanalartigen Formation (6,9) bei der reinen PUR-Lage (2) gleichen Materialtyps und gleicher Lagenstärke.

4. Ballonartige Struktur nach einem der vorhergehenden Ansprüche **dadurch gekennzeichnet, dass** wenigstens eine Lage (3) aus einem nicht elastischen Material, bspw. aus PVC, von einer plastischen, nicht-elastischen Beschaffenheit ist, so dass ihre flächige Verformung, Verbiegung oder Verwindung bei in situ wechselnden Fülldrucken des Ballons einen dämpfenden Effekt auf die Öffnungskinetik von ösen- bzw. kanalartigen Formationen (6,8) ausübt.

5. Ballonartige Struktur nach einem der vorhergehenden Ansprüche **dadurch gekennzeichnet, dass** die elastisch bedingte Öffnung bzw. Aufweitung der Ösen bzw. Kanäle bei passagerem oder zyklisch schwankendem Druckabfall im Ballon gegenüber einer einfach-lagigen, PVC-basierten ballonartigen Struktur verlangsamt ist.

6. Ballonartige Struktur nach einem der Ansprüche 3 bis 5, **dadurch gekennzeichnet, dass** der elastische Aufrichtungseffekt im Bereich der ösen- oder kanalartigen Umschlagformationen (6,8) reduziert ist, so dass die Querschnittsflächen der sekretleitenden, ösen- und kanalartigen Strukturen (6,8), im Gegensatz zu einer einfach-lagigen, elastischen Ballonfolie (2) nur aus PUR sowohl verkleinert sind als auch bei zyklischen Schwankungen des Ballonfülldrucks vermindert sind.

7. Ballonartige Struktur nach einem der vorhergehenden Ansprüche, **dadurch gekennzeichnet, dass** die gesamte Wandstärke der Ballonhülle (13) gleich oder kleiner ist als 50 µm, vorzugsweise gleich oder kleiner ist als 40 µm, insbesondere gleich oder kleiner ist als 30 µm.

8. Ballonartige Struktur nach einem der vorhergehenden Ansprüche **dadurch gekennzeichnet, dass** das Verhältnis der anteiligen Wandstärke der wenigstens einen PUR-Lage (2) zu der anteiligen Wandstärke der wenigstens einen Lage (3) aus einem nicht elastischen Material, bspw. aus PVC, zwischen 1 : 1 und 1 : 5 liegt, vorzugsweise zwischen 1 : 2 und 1 : 4 liegt, insbesondere etwa bei 1:3.

9. Ballonartige Struktur nach einem der vorhergehenden Ansprüche **dadurch gekennzeichnet, dass** wenigstens eine PUR-Lage (2) mit wenigstens einer PVC-Lage (3) kombiniert ist, so dass die Migration von Fluiden, insbesondere von polaren Flüssigkeiten, durch die Wandung (1) der Ballonhülle (13) reduziert ist gegenüber der Wandung (1) einer Ballonhülleaus reinem PUR.

10. Ballonartige Struktur nach einem der vorhergehenden Ansprüche **dadurch gekennzeichnet, dass** die ösen- oder kanalartigen Umschlagformationen (6,8) ihre Dichtungseigenschaft gegenüber Fluiden, insbesondere gegenüber Flüssigkeiten, beibehalten, solange der Ballonfülldruck und/oder die unteren Grenzwerte der zulässigen Schwankungen des Ballonfülldrucks bei oder oberhalb von 5 mbar liegen, vorzugsweise wobei sich die Querschnittsflächen der ösen- oder kanalartigen Umschlagformationen (6,8) um nicht mehr als 25 % erhöhen, vorzugsweise nur um 20 % oder weniger,
a) solange der Ballonfülldruck und/oder die unteren Grenzwerte der zulässigen Schwankungen des Ballonfülldrucks bei oder oberhalb von 5 mbar liegen, und/oder
b) solange die Druckamplitude und/oder die Differenz zwischen den beiden Druckextrema des Ballonfülldrucks in einem Bereich zwischen 5 mbar und 30 mbar liegt.

11. Ballonartige Struktur nach einem der vorhergehenden Ansprüche, **dadurch gekennzeichnet, dass** wenigstens eine Lage (3) aus einem nicht elastischen Material aus Polyvinylidenchlorid (PVDC) besteht, und/oder aus Ethylen-Vinylalkohol-Copolymer (EVOH).

12. Ballonartige Struktur nach einem der vorhergehenden Ansprüche **gekennzeichnet durch** eine dreilagige Ballonhülle.

13. Ballonartige Struktur nach einem der vorhergehenden Ansprüche, **dadurch gekennzeichnet, dass** zwischen der elastisch verformbaren PUR-Lage (2) und der nicht elastisch verformbaren Lage (3), beispielsweise aus PVC, eine gas- und/oder wasserdampfdichte Barriere-Lage (10), vorzugsweise aus PVDC oder EVOH, angeordnet ist, beispielsweise wobei die anteilige Wandstärke der nicht elastisch verformbaren Lage (3), beispielsweise aus PVC, größer ist als die anteiligen Wandstärken der elastisch verformbaren PUR-Lage (2) und/oder der gas- und/oder wasserdampfdichte Barriere-Lage (10), vorzugsweise aus PVDC oder EVOH, und/oder wobei vorzugsweise das Verhältnis zwischen der anteiligen Wandstärke der gas- und/oder wasserdampfdichten Barriere-Lage (10), vorzugsweise aus PVDC oder EVOH, und der anteiligen Wandstärke der nicht elastisch verformbaren Lage (3), bspw. PVC, zwischen 1 : 1 und 1 : 5 liegt, vorzugsweise zwischen 1 : 2 und 1 : 4, insbesondere etwa bei 1 : 3.

14. Ballonartige Struktur nach einem der vorhergehenden Ansprüche **dadurch gekennzeichnet, dass** die Ballonhülle (13) durch Blasformung eines mehrlagig extrudierten Schlauchrohlings hergestellt ist.

## Claims

1. Balloon-like structure having a balloon body and a balloon envelope, for positioning within a cavity in the human or animal body, for example within a lumen or some other interior space, in particular as an integral part of a catheter, in such a way that said cavity on the one hand is filled as completely as possible, i.e., without residual space, but on the other hand largely maintains its shape or is not deformed by the balloon body, whereby the balloon envelope (13) is made of a multi-layer balloon foil material, **characterized in that** at least one layer (2) is made from an elastically deformable polyurethane (PUR) and at least one other layer (3) is made from a non-elastic material such as polyvinyl chloride (PVC), whereby said at least one PUR layer (2) is made from thermoplastic PUR of a type having a water absorption of 5% or less according to the standard DIN ISO 62 in its version valid on the filing date (3^{rd} edition from the year 2008), preferably having a water absorption of 2% or less according to the standard DIN ISO 62 in its version valid on the filling date (3^{rd} edition from the year 2008), whereby the conjunction of several layers (2, 3, 10) of the balloon envelope (13) is obtained by co-extrusion of the several layers (2, 3, 10).

2. Balloon-like structure according to claim 1, **characterized in that** a layer (2) made of elastically deforming PUR material is'combined with a layer (3) made of plastically deforming material, in particular plastically deformable PVC.

3. Balloon-like structure according to claim 1 or 2, **characterized in that** typical invaginations of the excess residual balloon envelope, extending into the balloon interior, form upon the in situ placement of a residual balloon body, i.e., formed with excess balloon material along the circumference of the balloon, preferably wherein the invaginations extending into the balloon interior have cross-sectionally loop-like turned formations that preferably extend or continue as channel-like formations (8) in the longitudinal direction of the balloon, i.e., between its distal and proximal end-face sides (9), especially wherein at a filling pressure of the balloon of 30 mbar, the cross-sectionally loop-like turned formations (6) that preferably extend or continue as channel-like formations (8) in the longitudinal direction of the balloon have an opening diameter between 30 µm and 120 µm, preferably an opening diameter between 40 µm und 80 µm, for example wherein the opening diameter of the loop-like or channel-like formation (8) is reduced relative to the opening diameter of a loop-like or channel-like formation (6,8) at the pure PUR layer (2) of the same type of material and of the same thickness of the layer.

4. Balloon-like structure according to one of the preceding claims, **characterized in that** at least one layer (3) made of a nonelastic material, for example PVC, has a plastic, nonelastic quality such that its planar deformation, bending, or torsion under filling pressures of the balloon that change in situ exerts an attenuating effect on the opening kinetics of loop- or channel-like formations (6,8).

5. Balloon-like structure according to one of the preceding claims, **characterized in that** the elastically caused opening or, respectively, expansion of the loops or, respectively, channels upon a transient or cyclically fluctuating pressure drop in the balloon is slowed down in comparison with a one-layer PVC-based balloon-like structure.

6. Balloon-like structure according to one of the claims 3 to 5, **characterized in that** the elastic straightening effect in the region of the loop- or channel-like turned formations (6,8) is reduced in such a way that the cross-sectional areas of the secretion-conducting loop- and channel-like structures (6,8) are reduced in contrast to a single-layer elastic balloon film (2) made only of PUR, and are also decreased in the event of cyclical fluctuations of the balloon filling pressure.

7. Balloon-like structure according to one of the preceding claims, **characterized in that** the overall wall thickness of the balloon envelope (13) is equal to or less than 50 µm, preferably equal to or less than 40 µm, in particular equal to or less than to 30 µm.

8. Balloon-like structure according to one of the preceding claims, **characterized in that** the ratio of the proportional wall thickness of the at least one PUR layer (2) to the proportional wall thickness of the at least one layer (3) made of a nonelastic material, for example PVC, is between 1 : 1 and 1 : 5, preferably between 1 : 2 and 1 : 4, and in particular is approximately 1 : 3.

9. Balloon-like structure according to one of the preceding claims, **characterized in that** at least one PUR layer (2) is combined with at least one PVC layer (3), so that the migration of fluids, especially of polar liquids, through the wall (1) of the balloon envelope (13) is reduced in comparison with the wall (1) of the balloon envelope made from pure PUR.

10. Balloon-like structure according to one of the preceding claims, **characterized in that** the loop- or channel-like turned formations (6,8) maintain their sealing property with respect to fluids, in particular liquids, provided that the balloon filling pressure and/or the lower limit values of the allowed fluctuations of the balloon filling pressure is/are at or above 5 mbar, preferably wherein the cross-sectional areas of the loop- or channel-like turned formations (6, 8) increase by no more than 25%, preferably only by 20% or less,
a) as long as the balloon filling pressure and/or the lower limit values of the allowed fluctuations of the balloon filling pressure is/are at or above 5 mbar, and/or
b) as long as the pressure amplitude and/or the difference between the two pressure extremes of the balloon filling pressure are/is in a range between 5 mbar and 30 mbar

11. Balloon-like structure according to one of the preceding claims, **characterized in that** at least one layer (3) is made up of a nonelastic material made of polyvinylidene chloride (PVDC) and/or of ethylene vinyl alcohol (EVOH).

12. Balloon-like structure according to one of the preceding claims, **characterized by** a three-layer balloon envelope.

13. Balloon-like structure according to one of the preceding claims, **characterized in that** a gas- and/or water vapor-tight barrier layer (10), preferably made of PVDC or EVOH, is arranged between the elastically deformable PUR layer (2) and the nonelastically deformable layer (3) made of PVC, for instance, for example wherein the proportional wall thickness of the nonelastically deformable layer (3), made of PVC, for example, is greater than the proportional wall thicknesses of the elastically deformable PUR layer (2) and/or of the gas- and/or water vapor-tight barrier layer (10), preferably made of PVDC or EVOH, and/or wherein preferably the ratio of the proportional wall thickness of the gas- and/or water vapor-tight barrier layer (10), preferably made of PVDC or EVOH, to the proportional wall thickness of the nonelastically deformable layer (3), for example PVC, is between 1 : 1 and 1 : 5, preferably between 1 : 2 and 1 : 4, and in particular is approximately 1:3.

14. Balloon-like structure according to one of the preceding claims, **characterized in that** the balloon envelope (13) is manufactured by blow molding of a multilayer extruded tube blank.

## Revendications

1. Structure de type ballonnet avec un corps de ballonnet et une enveloppe de ballonnet (13) pour le positionnement à l'intérieur d'une cavité dans un corps humain ou animal, par exemple à l'intérieur d'une lumière ou d'un autre espace interne, en particulier sous forme de composant d'un cathéter, de telle sorte que ladite cavité est d'une part remplie le plus complètement possible, c'est-à-dire sans espace restant, mais tout en conservant autant que possible sa forme ou en n'étant pas déformée par le corps de ballonnet, en ce que l'enveloppe de ballonnet (13) est constituée d'un film de ballonnet multicouche, **caractérisée en ce qu'**au moins une couche (2) est constituée d'un polyuréthane (PUR) élastiquement déformable et au moins une autre couche (3) constituée d'un matériau non élastique comme par exemple le polychlorure de vinyle (PVC), **en ce qu'**au moins ladite couche PUR (2) est constituée d'un PUR thermoplastique de type présentant une absorption d'eau selon la norme DIN ISO 62 dans la version en vigueur à la date de dépôt : troisième édition de l'année 2008, de 5 % ou moins, de préférence d'une absorption d'eau selon la norme DIN ISO 62 dans la version en vigueur à la date de dépôt : troisième édition de l'année 2008, de 2 % ou moins, **en ce que** la liaison des différentes couches (2, 3, 10) de l'enveloppe de ballonnet (13) est réalisée par coextrusion des différentes couches (2, 3, 10).

2. Structure de type ballonnet selon la revendication 1, **caractérisée en ce qu'**une couche (2) en matériau PUR se déformant élastiquement est combinée avec une couche (3) en matériau se déformant plastiquement, en particulier en PVC plastiquement déformable.

3. Structure de type ballonnet selon la revendication 1 ou 2, **caractérisée en ce que** lors de la mise en place in situ d'un corps de ballonnet formé à une dimension résiduelle, c'est-à-dire avec un matériau de ballonnet excédentaire le long de la circonférence du ballonnet, il se forme des retournements invaginés typiques à l'intérieur du ballonnet de pli de ballonnet résiduelle excédentaire, de préférence **en ce que** le retournements invaginés à l'intérieur du ballonnet présentent en section transversale des formations d'enveloppe de type oeillet qui s'étendent ou se prolongent de préférence dans le sens longitudinal du ballonnet, donc entre sa face frontale (9) distale et proximale, sous forme de formations (8) de type canal, en particulier **en ce que** les formations pli (6) transversalement de type oeillet qui s'étendent ou se prolongent de préférence dans le sens longitudinal du ballonnet sous forme de formations (8) de type canal, présentent à une pression de remplissage du ballonnet de 30 mbar un diamètre d'ouverture compris entre 30 µm et 120 µm, de préférence un diamètre d'oeillet compris entre 40 µm et 80 µm, par exemple **en ce que** le diamètre d'ouverture de la formation (8) de type oeillet ou de type canal est réduit par rapport au diamètre d'ouverture d'une formation (6, 8) de type oeillet ou de type canal pour la couche PUR (2) pure de même type de matériau et de même épaisseur de couche.

4. Structure de type ballonnet selon l'une des revendications précédentes, **caractérisée en ce qu'**au moins une couche (3) en un matériau non élastique, par exemple en PVC, est de nature plastique, non élastique, de sorte que sa déformation plane, flexion ou torsion à des pressions de remplissage variables in situ du ballonnet exerce un effet amortissant sur la cinétique d'ouverture des formations de type oeillet ou canal (6, 8).

5. Structure de type ballonnet selon l'une des revendications précédentes, **caractérisée en ce que** l'ouverture élastiquement restreinte ou élargissement des oeillets ou canaux en cas de chute de pression variable de manière passagère ou cycliquement dans le ballonnet par rapport à une structure de type ballonnet à une couche sur une base PVC est ralentie.

6. Structure de type ballonnet selon l'une des revendications 3 à 5, **caractérisée en ce que** l'effet de redressement élastique au niveau des formations d'enveloppe de type oeillet ou canal (6, 8) est réduit, de sorte que les surfaces de section des structures conductrices de secrétions, de type oeillet et canal (6, 8), au contraire d'un film de ballonnet (2) élastique à une couche, constitué uniquement de PUR, sont aussi bien réduites que diminuées également en cas de variations cycliques de la pression de remplissage du ballonnet.

7. Structure de type ballonnet selon l'une des revendications précédentes, **caractérisée en ce que** l'épaisseur totale de la paroi de l'enveloppe de ballonnet (13) est égale ou inférieure à 50 µm, de préférence égale ou inférieure à 40 µm, en particulier égale ou inférieure à 30 µm.

8. Structure de type ballonnet selon l'une des revendications précédentes, **caractérisée en ce que** le rapport de l'épaisseur de paroi proportionnelle d'au moins une couche PUR (2) par rapport à l'épaisseur de paroi proportionnelle d'au moins une couche (3) constituée d'un matériau non élastique, par exemple constituée de PVC, est de 1:1 et 1:5, de préférence entre 1:2 et 1:4, en particulier approximativement de 1:3.

9. Structure de type ballonnet selon l'une des revendications précédentes, **caractérisée en ce qu'**au moins une couche PUR (2) est combinée avec au moins une couche PVC (3), de sorte que la migration de fluides, en particulier de liquides polaires, est réduite par la paroi (1) de l'enveloppe de ballonnet.(13) par rapport à la paroi (1) d'une enveloppe de ballonnet en PUR pur.

10. Structure de type ballonnet selon l'une des revendications précédentes, **caractérisée en ce que** les formations pli de type oeillet ou canal (6, 8) conservent leur caractéristique d'étanchéité par rapport aux fluides, en particulier par rapport aux liquides, tant que la pression de remplissage du ballonnet et/ou les valeurs limites inférieures des variations admissibles de la pression de remplissage du ballonnet sont égales ou au-dessus de 5 mbar, de préférence **en ce que** les surfaces de section des formations pli de type oeillet ou canal (6, 8) n'augmentent pas plus de 25 %, de préférence de seulement 20% ou moins,
a) tant que la pression de remplissage du ballonnet et/ou les valeurs limites inférieures des variations admissibles de la pression de remplissage du ballonnet sont égales ou au-dessus de 5 mbar, et/ou
b) tant que l'amplitude de pression et/ou la différence entre les deux extrêmes de pression de la pression de remplissage du ballonnet sont dans une plage comprise entre 5 mbar et 30 mbar.

11. Structure de type ballonnet selon l'une des revendications précédentes, **caractérisée en ce qu'**au moins une couche (3) est constituée d'un matériau non élastique en polychlorure de vinylidène (PVDC), et/ou en copolymère éthylène/alcool vinylique (EVOH).

12. Structure de type ballonnet selon l'une des revendications précédentes, **caractérisée par** une enveloppe de ballonnet à trois couches.

13. Structure de type ballonnet selon l'une des revendications précédentes, **caractérisée en ce qu'**entre la couche PUR (2) élastiquement déformable et la couche (3) non élastiquement déformable, par exemple en PVC, est disposée une couche barrière (10) étanche au gaz et/ou à la vapeur d'eau, de préférence en PVDC ou EVOH, par exemple **en ce que** l'épaisseur de paroi proportionnelle de la couche (3) non élastiquement déformable, par exemple en PVC, est supérieure aux épaisseurs de parois proportionnelles de la couche PUR (2) élastiquement déformable et/ou de la couche barrière (10) étanche au gaz et/ou à la vapeur d'eau, de préférence en PVDC ou EVOH, et/ou **en ce que** de préférence le rapport entre l'épaisseur de paroi proportionnelle de la couche barrière (10) étanche au gaz et/ou à la vapeur d'eau, de préférence en PVDC ou EVOH, et l'épaisseur de paroi proportionnelle de la couche (3) non élastiquement déformable, par exemple en PVC, est entre 1:1 et 1:5, de préférence entre 1:2 et 1:4, en particulier approximativement de 1:3.

14. Structure de type ballonnet selon l'une des revendications précédentes, **caractérisée en ce que** l'enveloppe de ballonnet (13) est réalisée par moulage par soufflage d'une ébauche de tuyau extrudée multicouche.
